# EUROPEAN PATENT APPLICATION

(11) **EP 4 782 505 A1**
(43) Date of publication of application: **29.07.2026**
(21) Application number: 25153363.4
(22) Date of filing: 22.01.2025
(51) Int. Cl.: C09B 57/00, C07D 305/14, G01N 33/58

(54) **FUNCTIONALIZED DOCETAXEL PROBES FOR LABELING MICROTUBULES**

(71) Applicant: Julius-Maximilians-Universität Würzburg, in Vertretung des Freistaates Bayern, 97070 Würzburg (DE)
(72) Inventor: SAUER, Markus, 97074 Würzburg (DE); WEN, Gang, 97074 Würzburg (DE)
(74) Representative: Kador & Partner Part mbB

(57) **Abstract**

The present invention relates to a compound for labeling microtubules. The compound is a taxane derivative comprising at least on primary amino group and a group X suitable for covalently linking the compound to form a conjugate with a labeling compound. The present invention also relates to a conjugate formed by a reaction of the inventive compound with a labeling compound comprising a group Y capable of reacting with group X of the inventive compound, to a method for producing the inventive compound, a use of the inventive compound or conjugate in a method for labeling microtubules, and a kit of parts comprising the inventive compound and a labeling compound.

The compound for labeling microtubules has formula (I): 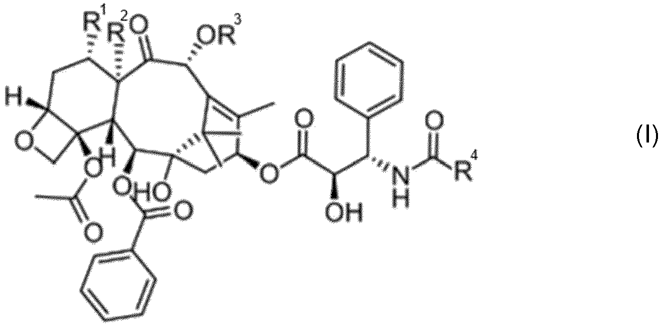 wherein R4 is represented by formula (III): 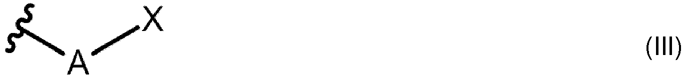 wherein A is a C1 to C25 alkyl substituted with at least one primary amino-group and one or more carbon atoms of the alkyl chain optionally being replaced by an amide group; and

wherein X is a group suitable for covalently linking the compound to form a conjugate with a labeling compound.

## Description

The present invention relates to a compound for labeling microtubules, a conjugate formed by a reaction of said compound with a labeling compound, a method for producing said compound, a use of said compound in a method for labeling microtubules and a kit of parts for labeling microtubules comprising said compound.

### DESCRIPTION

Fluorescent taxane probes, such as docetaxel, paclitaxel, cabazitaxel or larotaxel probes, are a well-known tool to label microtubules for imaging in living cells. However, the known methods and previously used derivatives of taxanes have some major drawbacks, such as cytotoxicity at higher concentrations due to their inhibitory effect on microtubule dynamics. Furthermore, previously used taxane derivatives and labeling methods using known derivatives are disadvantageous, since these compounds are not fixable and washed out during fixation of the cells. Therefore, the taxane derivatives known in the art are not useable for all imaging techniques. Particularly, the use of known probes excludes imaging techniques that can only be performed with fixed substrates. For example, Expansion microscopy (ExM) which is an approach to achieve super-resolution imaging via physical expansion of biological samples is not compatible with common probes. Another issue of known probes in this regard is their lack of anchorable groups for covalent crosslinking into polyacrylamide hydrogels during expansion resulting in washing out of the fluorescence signal.

Alternative methods for staining microtubules involve the use of antibodies. However, particularly referring to staining of microtubules for imaging microtubules for Expansion microscopy, only poor expansion factors could be achieved due to low and inhomogenous labeling densities.

It is an object of the present invention to overcome the above issues related to imaging of microtubules and provide a compound for labeling microtubules with improved characteristics, avoiding the above issues.

Furthermore, it is a particular object of the present invention to provide probes that are usable in Expansion microscopy which is an approach to achieve super-resolution imaging via physical expansion of biological samples and therefore suitable for resolving dense structures.

In a first aspect, the present invention relates to a compound for labeling microtubules according to formula (I) 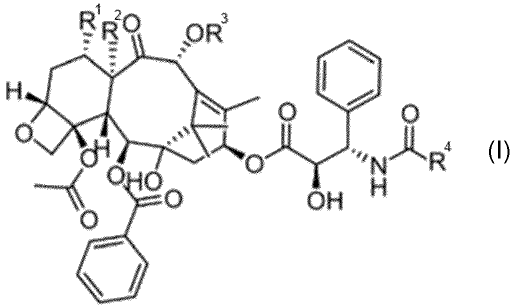 wherein R¹ represents a hydroxyl group, R² represents a methyl group and R³ represents a hydrogen atom or an acetyl group; or R¹ represents a methoxy group and R² and R³ represent a methyl group; or R¹ and R² are represented by formula (II) and R³ represents an acetyl group; and 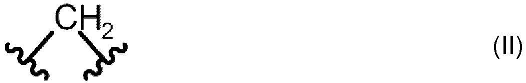 wherein R⁴ is represented by formula (III) 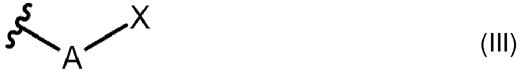 wherein A is a C1 to C25 alkyl substituted with at least one primary amino-group and one or more carbon atoms of the alkyl chain optionally being replaced by an amide group; and wherein group X is a group suitable for covalently linking the compound to form a conjugate with a labeling compound.

The length of the substituted alkyl may be from 3 to 20 carbon atoms, from 5 to 17 carbon atoms, from 7 to 15, from 9 to 13 or from 10 to 12 carbon atoms, wherein one or more carbon atoms are optionally replaced by an amide group.

Group A is substituted with at least one primary amino-group. The at least one or any further carbon atom substituted with the primary amino-group of group A is preferably separated from group X by at least 2, more preferably at least 3, carbon atoms of the alkyl chain.

Preferably, R¹ represents a hydroxyl group, R² represents a methyl group and R³ represents a hydrogen atom.

The inventive compound according to formula (I) may herein be further referred to as compound (I).

Group R⁴ of the inventive compound eliminates two major problems of known docetaxel-based probes for the usability for labeling microtubules. First, the problem of conventional docetaxel-based fluorescent probes to be easily washed out during fixation of a substrate that has been stained with such a probe is solved, particularly due to the addition of the primary amino-group. Therefore, the inventive compound may be efficiently applied in sample preparation for imaging techniques that are to be performed on fixed samples such as Expansion microscopy.

Addition of at least one functional group X being a group suitable for covalently linking the compound to form a conjugate with a labeling compound allows a better accessibility to microtubules of the probe than for directly fluorophore-coupled docetaxel derivatives. Probably due to the small size of the functional groups introduced, there are no or only minor alterations of the binding efficiency of the inventive compound compared to conventionally used docetaxel derivatives.

In addition, depending on the requirements of a particular experimental approach, conjugates formed by reaction of the inventive compound and the labeling compound may be generated prior to contacting the inventive compound with the sample or in situ, when the inventive compound is already bound to the microtubules and the sample is fixed.

In preferred embodiments, A is represented by formula (IV) 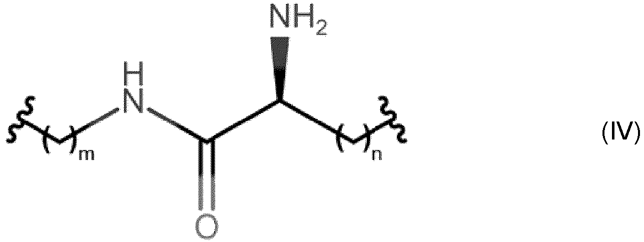 wherein m and n are numbers independently selected from the range of 1 to 10. m is preferably from 3 to 7, more preferably from 4 to 6 most preferably 5. n is preferably from 2 to 10, more preferably from 2 to 8, such as from 2 to 7, 3 to 6 or 4 to 5, most preferably 4. In a most preferred embodiment, m is 5 and n is 4.

In the sense of the present invention, a group X suitable for covalently linking the compound to form a conjugate with a labeling compound is a functional group that is commonly applied as reacting group in reactions for covalently linking two compounds within the concept of "click chemistry". Such reactions are characterized by features such as high yield, modular and broad applicability, simplicity, and high specificity. For example, such reactions can be [3+2] cycloaddition reactions, such as the Huisgen 1,3-dipolar cycloaddition, thiol-ene reactions, Diels-Alder reaction, [4+1] cycloadditions, and nucleophilic substitution to small strained rings, such as epoxy or aziridine groups.

Group X may be an azido-group, alkynyl-group, thiol-group, alkenyl-group, a conjugated diene group, epoxy-group, aziridine group, tetrazine group, or TCO (trans-cyclooctene)-group or derivative thereof.

Preferably, group X is an azido-group or an alkynyl-group. More preferably, group X is an azido-group.

In an exemplary embodiment, group A is represented by formula (IV), wherein m is 5 and n is 4, and X is an azido-group.

In a most preferred exemplary embodiment, R¹ represents a hydroxyl group, R² represents a methyl group and R³ represents a hydrogen atom, and A is represented by formula (IV), wherein m is 5 and n is 4, and X is an azido-group. The compound of said most preferred embodiment is shown by formula (V).

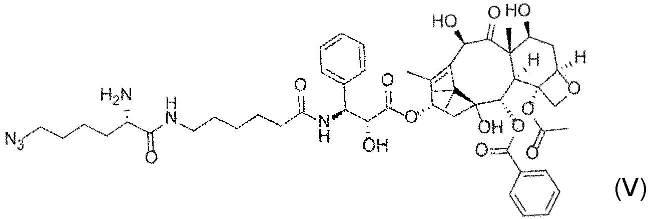

In a second aspect, the present invention relates to a conjugate formed by a reaction of the inventive compound described above with a labeling compound comprising a group Y capable of reacting with group X of the inventive compound.

In other words, group X of the inventive compound according to formula (I) reacts with group Y of the labeling compound to form said conjugate. Therefore, group Y is selected to be a counterpart of group X in a reaction within the concept of click chemistry. Exemplary suitable pairs of group X and group Y may be selected from the group of pairs consisting of azido-group and alkynyl-group, alkynyl-group and azido-group, thiol-group and alkenyl-group, alkenyl-group and thiol-group, conjugated diene-group and alkynyl-group, alkynyl-group and conjugated diene-group, TCO-group or derivative thereof and tetrazine-group, tetrazine-group and TCO-group or derivative thereof, epoxy-group or aziridine group and nucleophilic group, such as an amino group, or nucleophilic group and epoxy-group or aziridine group.

In preferred embodiments, group X may be an azido-group and group Y an alkynyl-group or group X may be an alkynyl-group and group Y an azido-group. More preferably, group X is an azido-group and group Y is an alkynyl group.

In a particular exemplary embodiment, the conjugate is formed by a reaction of the compound according to formula (V) with a labeling compound comprising an alkynyl-group as group Y.

If group Y is an alkynyl-group, the alkynyl-group may be comprised in a tag, such as a dibenzocyclooctyne (DBCO)-tag.

In some embodiments, the labeling compound is a fluorophore functionalized with the group Y

The application of fluorophores is not limited. The use of functionalized fluorophores as labeling compound may for example be applied, when the compound or conjugate are used for labeling microtubules for detection by high resolution imaging, such as structured illumination microscopy (SIM), airyscan microscopy and dSTORM microscopy. In such applications, the inventive compound in combination with a labeling compound or the inventive conjugate can be advantageously characterized by a small linkage error.

In other embodiments, the labeling compound is an affinity-tag suitable to be further labeled by proteins or antibodies, which are targeted to the affinity tag, and functionalized with the group Y. The affinity-tag may be a small molecule or short peptide tag. A short peptide tag as used herein can be any peptide sequence that can be labeled with proteins and is within a length of 5 to 10 amino acids, such as 5, 6, 7, 8, 9 or 10 amino acids.

A labeling compound comprising a small molecule can be biotin functionalized with group Y A labeling compound comprising a short peptide tag can be a Strep-tag or a HA-tag functionalized with the group Y. Within such embodiments, it is preferred that the labeling compound is biotin functionalized with group Y. More preferably, the labeling compound is biotin functionalized with an azido- or alkynyl-group, most preferably an alkynyl-group, as group Y.

The affinity tag is suitable to be further labeled by fluorophore-conjugated proteins targeted to the tag. Such proteins can be for example streptavidin, if the labeling compound comprises biotin, or antibodies.

A labeling compound in the form of affinity-tags functionalized with group Y, as previously specified, may for example be applied, when the compound or conjugate are used for labeling microtubules for detection by Expansion microscopy. It is particularly preferred to use biotin functionalized with group Y This enables processing of the sample for detection of microtubules by Expansion microscopy. The conjugate formed by a reaction of the inventive compound and a biotin-containing labeling compound enables staining with fluorescently labeled streptavidin and streptavidin enables efficient grafting of the conjugate into the polymer matrix, followed by digestion and expansion.

Among embodiments, wherein the labeling compound comprises an affinity tag, the conjugate is most preferably formed by a reaction of the compound according to formula (V) with a labeling compound being biotin functionalized with an alkynyl-group as group Y.

Which labeling compound functionalized with group Y is chosen in a particular embodiment, is in principle not limited but may depend on the question which method is chosen for detecting the conjugate in a sample labeled by using the inventive compound or conjugate.

In a third aspect, the present invention relates to a method for producing the inventive compound described above. Therein, docetaxel, paclitaxel, cabazitaxel or larotaxel is derivatized to the inventive compound.

Docetaxel may be derivatized to the inventive compound according to formula (I), wherein R¹ represents a hydroxyl group, R² represents a methyl group and R³ represents a hydrogen atom. Paclitaxel may be derivatized to the inventive compound according to formula (I), wherein R¹ represents a hydroxyl group, R² represents a methyl group and R³ represents an acetyl group. Cabazitaxel may be derivatized to the inventive compound according to formula (I), wherein R¹ represents a methoxy group and both R² and R³ represent a methyl group. Larotaxel may be derivatized to the inventive compound according to formula (I), wherein R¹ and R² are represented by formula (II) and R³ represents an acetyl group.

Preferred embodiments of the inventive compound equally apply to the method for producing the compound.

In a fourth aspect, the present invention relates to a use of the inventive compound or conjugate in a method for labeling microtubules.

The conjugate is preferably formed in situ. This is particularly advantageous, since the inventive compound is a docetaxel derivative having introduced only functional groups of small size. Hence, the introduction of such small functional groups causes no or only minor alterations of the binding efficiency of the inventive compound to microtubules compared to docetaxel.

Preferably, the compound is used in a method for labeling microtubules comprising the steps of (I) contacting a sample with the inventive compound to allow binding of said compound to the microtubules; and (II) contacting the sample having said compound bound to the microtubules with the labeling compound, wherein the conjugate is formed by a reaction of group X with group Y of the labeling compound.

The samples, as referred to herein, may be eukaryotic or prokaryotic cells, preferably eukaryotic cells, for example eukaryotic primary cells or cell lines.

The use, in particular embodiments, relates to a method for labeling microtubules of mitotic spindles, preferably a method for labeling microtubules throughout the whole cell cycle.

The method may further comprise a step of treating the sample with a fixing agent, preferably after step (I). The fixing agent is preferably an aldehyde-based fixing agent, such as formaldehyde, formalin, paraformaldehyde, or glutaraldehyde, more preferably glutaraldehyde.

In a particular embodiment of the inventive use, the labeling compound is an affinity-tag suitable to be further labeled by proteins or antibodies, preferably biotin, and is functionalized with the group Y. In such embodiments, the method may further comprise (III) contacting the sample with a fluorophore-conjugated protein targeted to the labeling compound; and (IV) optionally further processing the sample for detecting the conjugate by Expansion microscopy. Steps (III) and optionally (IV) may be carried out after step (II). In said particular embodiment, it may be preferable to treat the sample with a fixing agent as defined above and/or that the labeling compound is biotin functionalized with group Y and the protein targeted to the labeling compound is fluorophore-conjugated streptavidin.

The advantageous characteristics of the inventive compound and conjugate as set forth above are equally applicable with regard to the use of said compound and/or conjugate in the method for labeling microtubules.

In particular embodiments, the use may relate to methods wherein the sample is only subjected to labeling of microtubules using the inventive compound and/or conjugate. However, the inventive compound and/or conjugate, when used in a method for labeling microtubules, is compatible with co-labeling of the sample.

Hence, in specific embodiments, the use may relate to methods, wherein the sample is subjected to labeling of microtubules using the inventive compound and/or conjugate and is co-stained with other compounds. Such other compounds may be suitable to label other cellular components, such as the nucleus, DNA, mitochondria or other cytoskeletal components.

The method for detecting the conjugate bound to microtubules is not particularly limited. A super-resolution imaging method may be applied, such as direct Stochastic Optical Reconstruction Microscopy (dSTORM), airyscan microscopy, or Structured Illumination Microscopy (SIM). In some embodiments, Expansion Microscopy (ExM) may be applied. The choice of the method for detecting the conjugate may, however, be limited by the particular embodiment of the method chosen for labeling microtubules and by the choice of the labeling compound, as described above.

Due to the high specificity, labeling efficiency and binding density to microtubules, a high resolution can be achieved, wherein the inventive compound or conjugate is used for labeling microtubules, e.g. for labeling microtubules in mitotic spindles throughout all stages of mitosis.

In a fifth aspect the present invention relates to a kit of parts comprising the inventive compound and the labeling compound, wherein the labeling compound comprises a group Y capable of reacting with group X of compound (I) to form a conjugate.

The kit of parts according to the invention may further comprise a fixing agent, preferably an aldehyde-based fixing agent, such as formaldehyde/formalin, paraformaldehyde, or glutaraldehyde, most preferably glutaraldehyde.

Features and preferred embodiments described herein for the inventive compound, the conjugate, the method for producing the compound, the use of the compound and the kit of parts comprising the compounds, may apply equally as preferred embodiments of the respective other aspects of the invention.

### BRIEF DESCRIPTION OF THE FIGURES

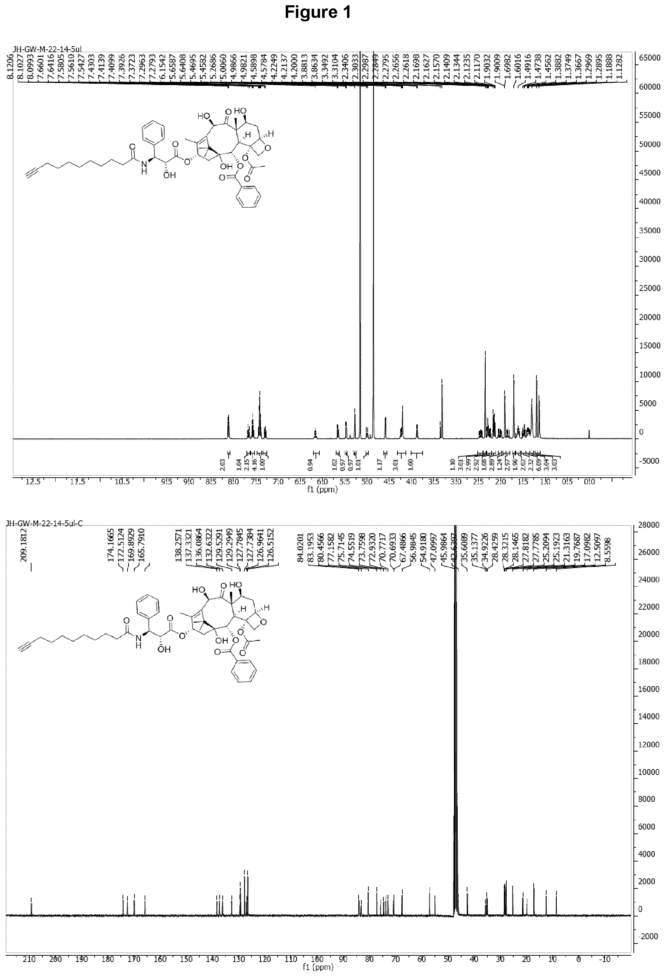

Figure 1: ¹H and ¹³C NMR spectra of compound 1a (Comparative example 3).

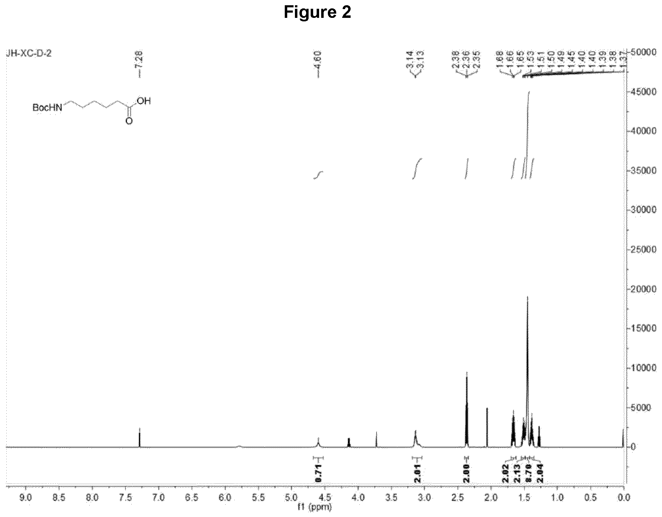

Figure 2: ¹H NMR spectrum of compound S2 (intermediate in the synthesis of compound 1b (Inventive example 1)).

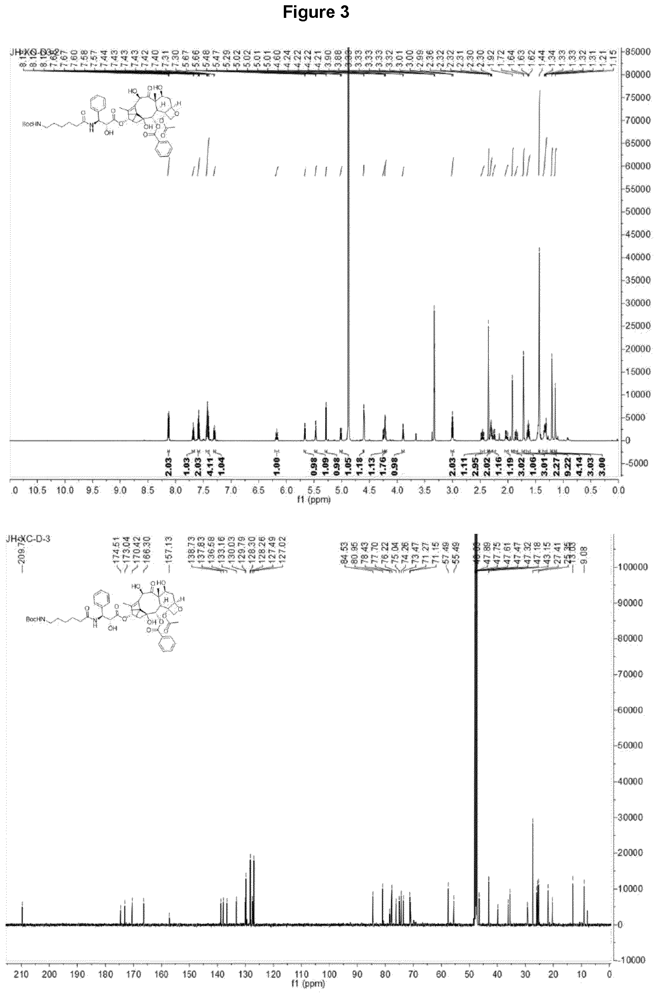

Figure 3: ¹H and ¹³C NMR spectra of compound S3 (intermediate in the synthesis of compound 1b (Inventive example 1)).

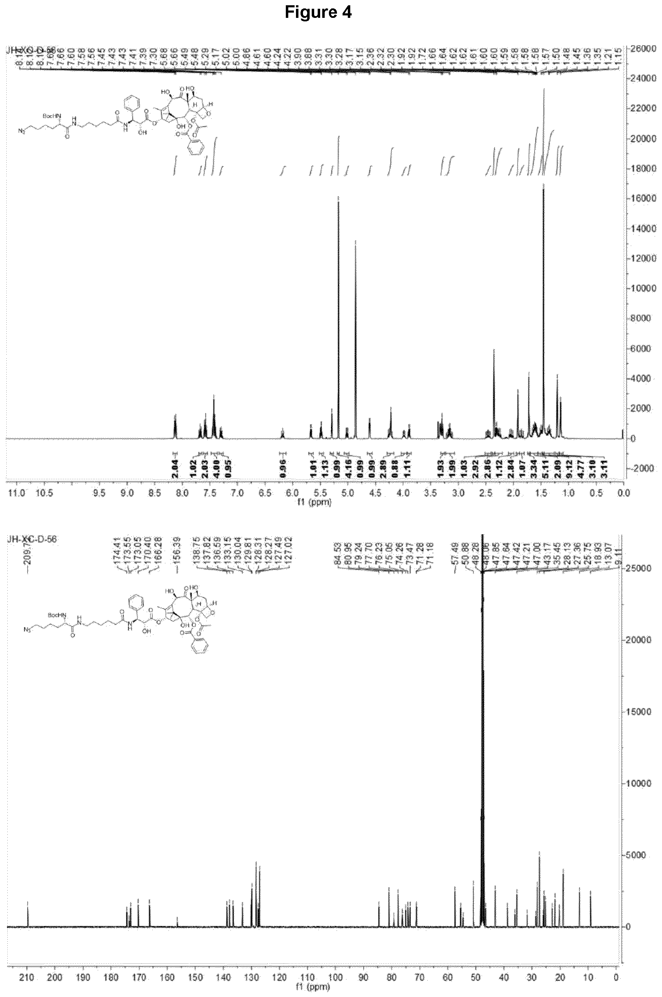

Figure 4: ¹H and ¹³C NMR spectra of compound S5 (intermediate in the synthesis of compound 1b (Inventive example 1)).

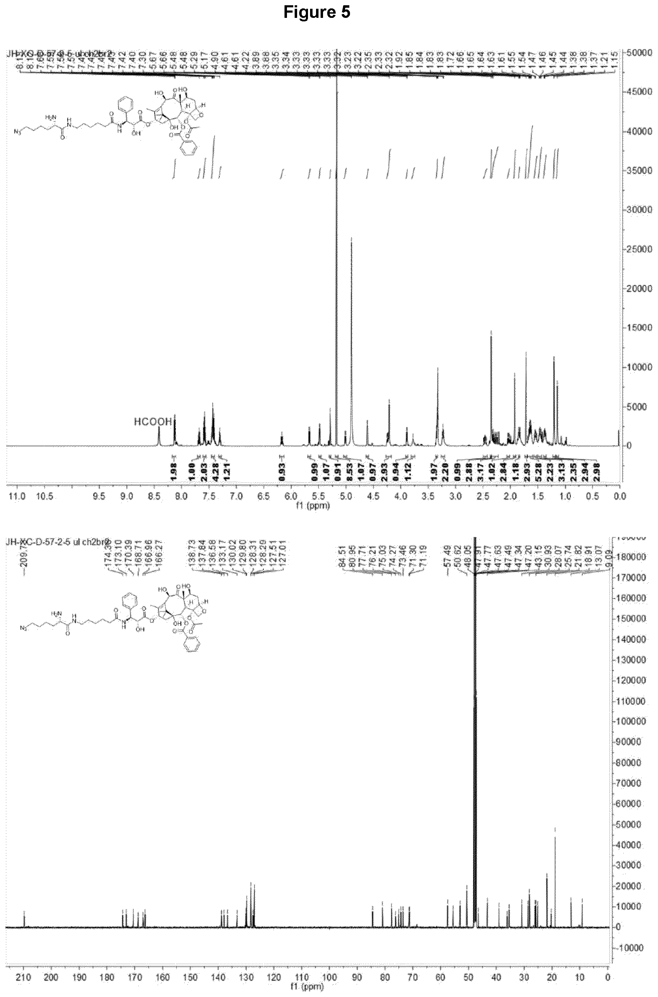

Figure 5: ¹H and ¹³C NMR spectra of compound 1b (Inventive example 1).

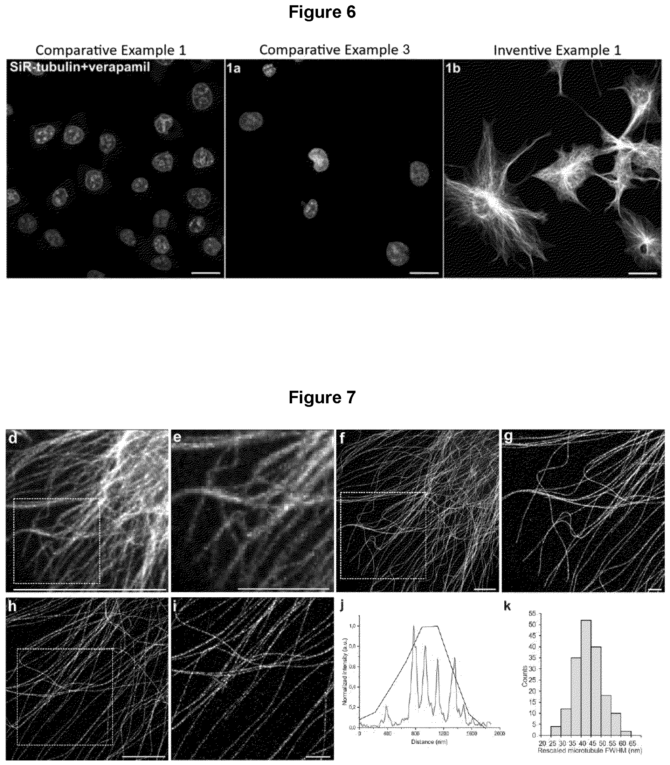

Figure 6: Comparison of signal of microtubules in fixed cells. Cells were incubated with the indicated probes according to comparative examples 1 (SiR-tubulin) and 3 (compound 1a) and inventive example 1 (compound 1b) for 1 h, followed by fixation with 0.5% GA and permeabilization with Triton X-100. ATTO 643-Azide and ATTO 643-DBCO were used to introduce fluorescence in samples labeled with compound 1a (comparative example 3) or compound 1b (inventive example 1), respectively. DNA was stained with DAPI. All images were acquired with the same parameters.
Figure 7: After labeling microtubules with compound 1b of inventive example 1 as shown in Figure 6, cells are fixed, permeabilized, reacted with DBCO-PEG4-biotin and stained with streptavidin-dyes, followed by the procedure of ExM. d-g, Comparison of confocal images of the same microtubules before (d,e) and after TREx (f,g). h,i, Airyscan SR image of the same cell after TREx (h) and zoom-in views of the highlighted region (i). j, Fluorescence intensity profiles along the red lines in e ad i. k, Distribution of Gaussian-fitted full width at half-maximum (FWHM) of microtubule intensity profiles, yielding a resolution of 43.64 ± 7.14 nm (mean ± s.d., n= 173). Representative images were recorded from n>4 **(b)** or 4 **(d-i)** independent samples. Scale bars, **b,d,f,h** 30 µm; **e,g,i** 10 µm.

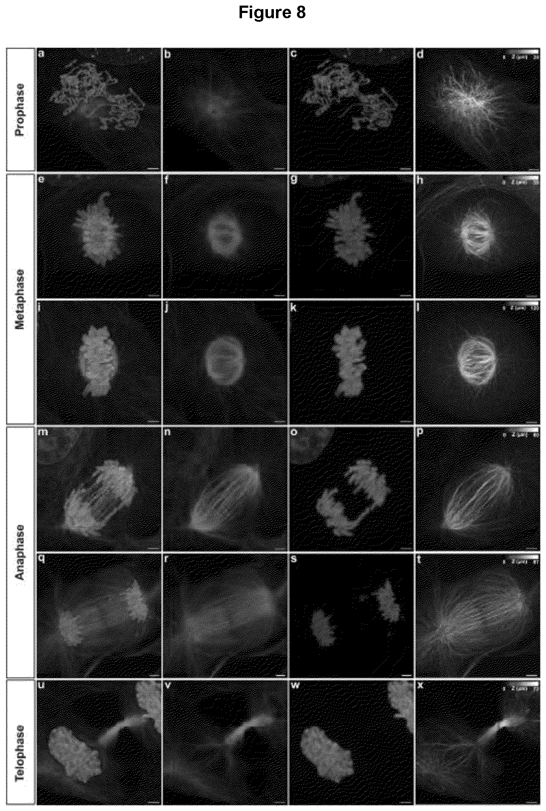

Figure 8: Super-resolution images of TREx -expanded microtubules at different mitotic stages. Microtubules in mitotic COS-7 cells were labeled with compound 1b (inventive example 1), fixed, permeabilized, click-labeled with DBCO-biotin, and stained with streptavidin-CF568, followed by expansion using the TREx protocol. DNA was stained with DAPI. a-d, Two-color images of microtubules (red) and nuclei (cyan) at prophase (a-c) and corresponding 3D microtubule imaging (d). e-l, Two-color images of microtubules (red) and nuclei (cyan) at metaphase (e-g, i-k) and corresponding 3D microtubule imaging (h,l). m-t, Two-color images of microtubules (red) and nuclei (cyan) at anaphase (m-o, q-s) and corresponding 3D microtubule imaging (p,t). u-x, Two-color images of microtubules (red) and nuclei (cyan) at telophase (u-w) and corresponding 3D microtubule imaging (x). The z-position is indicated by the color coding. Representative images were from three independent samples. Scale bars, 20 µm.

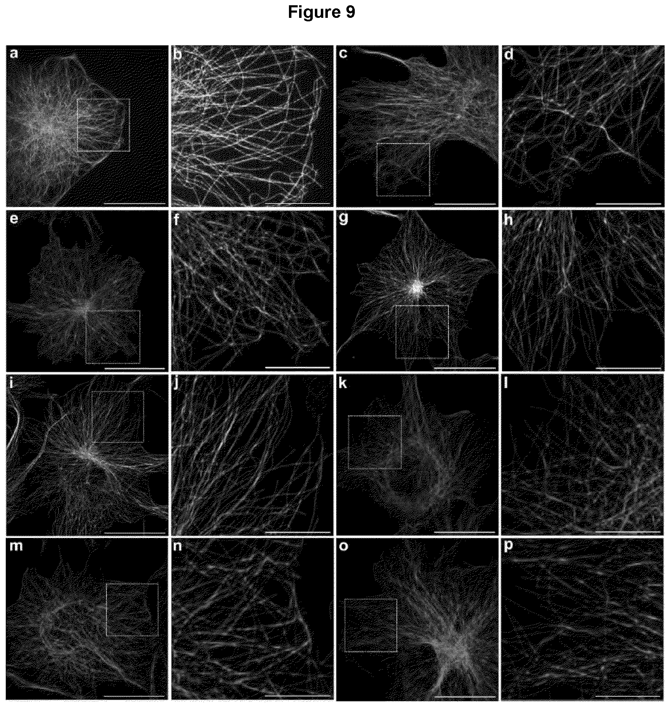

Figure 9: SIM image of microtubules in COS-7 cells stained with compound 1b (inventive example 1) and click-labeled with various organic dyes. Cells fed with compound 1b were fixed, permeabilized and stained with different DBCO-modified organic dyes. a,b, SIM image of microtubules with Pacific blue. c,d, SIM image of microtubules with Bodipy FL. e,f, SIM image of microtubules with Alexa Flour 488. g,h, SIM image of microtubules with Rhodamine B. i.j. SIM image of microtubules with Alexa Fluor 568. k,l, SIM image of microtubules with Alexa Flour 594. m,n, SIM image of microtubules with Alexa Fluor 647. o,p, SIM image of microtubules with ATTO 643. Representative images were from three independent samples. Scale bars, a,c,e,g,i,k,m,o 30 µm; b,d,f,h,j,l,n,p 10 µm.

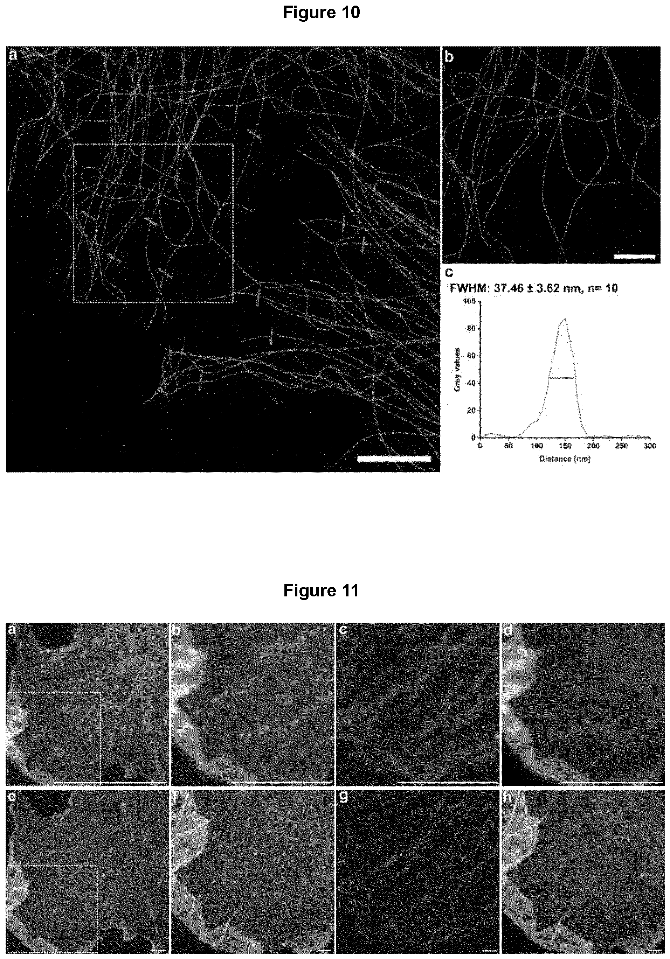

Figure 10: dSTORM image of microtubules in COS-7 cells using compound 1b (inventive example 1). After fixation and permeabilization, compound 1b-labeled microtubules were stained with AF647-DBCO. a,b, dSTORM image of microtubules stained with AF647 (a) and the magnified view (b). c, Analysis of cross-sectional intensity profile of 10 microtubule profiles shown in a (red lines). Representative images were taken from two independent samples. Scale bars, a 5 µm; b 2 µm.
Figure 11: Multicolor images of microtubules labeled with compound 1b (inventive example 1) and actin filaments stained with phalloidin in COS-7 cells using TREx. a, Pre-expansion confocal fluorescence image of microtubules visualized with compound 1b (ATTO 643, magenta) or actin filaments (`Actin ExM', 561, green). b, Magnified views of the boxed region in panel a. c,d Magenta and green channel in b. e, Post-expansion confocal fluorescence image of microtubules obtained after TREx in the same cell. f, Magnified views of the boxed region in e. g.h, Magenta and green channel in f. Representative images were from three independent samples. Scale bars, a,e 20 µm; b-d,f-h 10 µm.

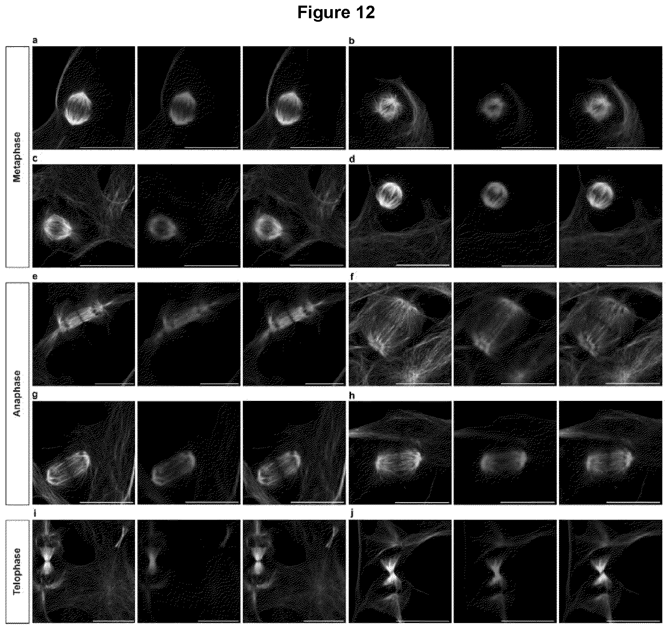

Figure 12: Comparison of microtubules labeled in COS-7 cells with compound 1b (inventive example 1) and anti-β tubulin antibodies (comparative example 2a) at different cell cycle stages by airyscan microscopy. a-d, Two color images of microtubules stained with compound 1b (CF 568, red) and anti-β tubulin antibodies (AF 488, green) at metaphase. e-h, Two color images of microtubules stained with compound 1b (CF 568, red) and anti-β tubulin antibodies (AF 488, green) at anaphase. i,j, Two color images of microtubules stained with compound 1b (CF 568, red) and anti-β tubulin antibodies (AF 488, green) at telophase. Representative images were from n=3 independent samples. Scale bars, 20 µm.

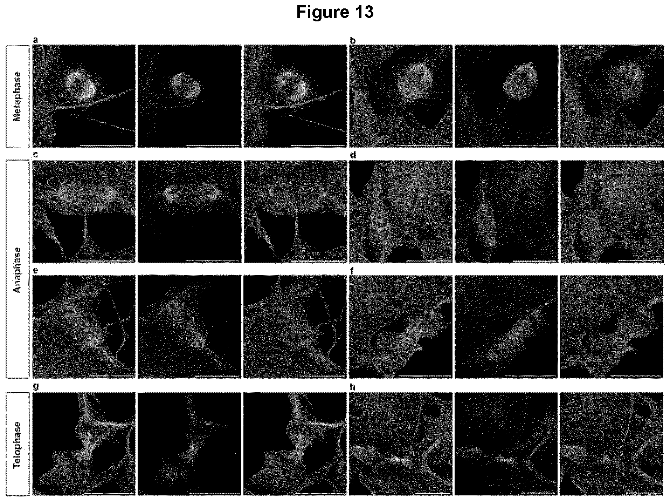

Figure 13: Comparison of microtubules labeled in COS-7 cells with compound 1b (inventive example 1) and anti-α tubulin antibodies (comparative example 2b) at different cell cycle stages by airyscan microscopy. a,b, Two color images of microtubules stained with compound 1b (CF 568, red) and anti-α tubulin antibodies (AF 488, green) at metaphase. c-f, Two color images of microtubules stained with compound 1b (CF 568, red) and anti-α tubulin antibodies (AF 488, green) at anaphase. g,h, Two color images of microtubules stained with compound 1b (CF 568, red) and anti-α tubulin antibodies (AF 488, green) at telophase. Representative images were from n=2 independent samples. Scale bars, 20 µm.

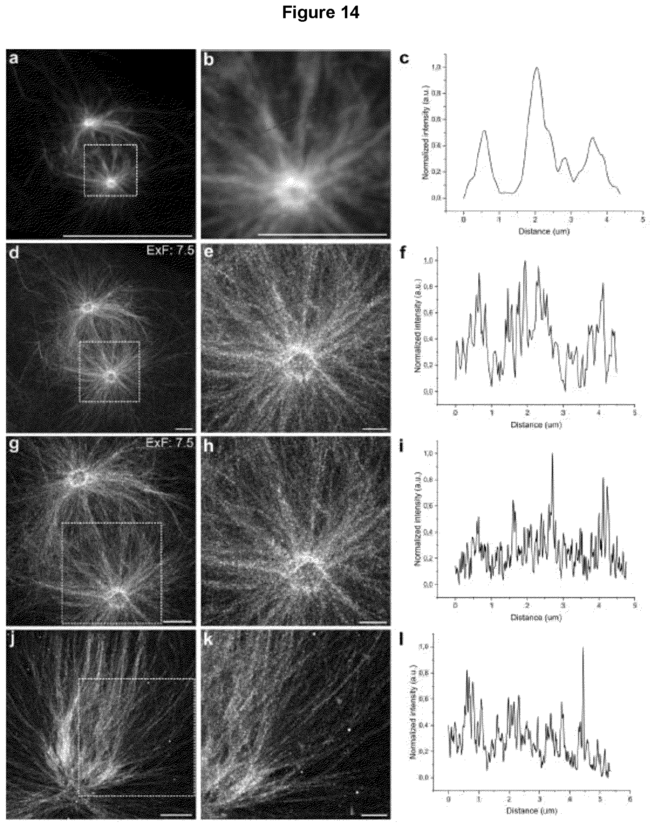

Figure 14: Comparison of super-resolution images of microtubules at mitotic states before and after TREx. a, Pre-expansion airyscan image of microtubules. b, Magnified views of the boxed region in a. c, Fluorescence intensity profiles along the purple dotted lines in b. d, Post-expansion confocal image of microtubules in the same cell. e, Magnified views of the boxed region in d. f, Fluorescence intensity profiles along the purple dotted lines in e. The distance is corresponding to pre-expansion dimension based on expansion factor. g, Airyscan SR image of the same cell after TREx. h, Zoom-in views of the highlighted region in g. The red arrowheads point at individual microtubules within microtubule bundles. i, Fluorescence intensity profiles along the purple dotted line in h. The distance is corresponding to pre-expansion dimension based on expansion factor. j, Airyscan SR image of microtubules in the anaphase cell after TREx. k, Zoom-in views of the highlighted region in j. The red arrowheads point at individual microtubules within microtubule bundles. l, Fluorescence intensity profiles along the purple dotted line in k. In f,i,l, the distance is corresponding to pre-expansion dimension based on expansion factor. Representative images were from three independent samples. Scale bars, a,d,g,j 20 µm; e,h,k 10 µm; b 5 µm.

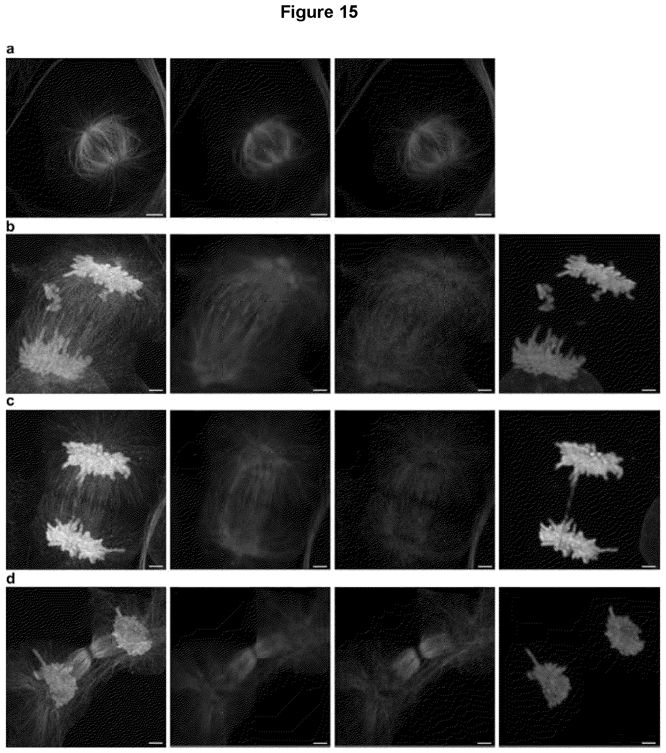

Figure 15: Comparison of TREx-expanded microtubules in mitotic COS-7 cells using compound 1b (inventive example 1) and antibodies to β tubulin (comparative example 2a). Microtubules stained with compound 1b (CF568, red) or with anti-β tubulin antibodies (AF 488, green) and nuclei stained with DAPI (cyan) are shown, a, Two-color images of microtubules in metaphase cells. b,c, Three-color images of microtubules and nuclei in anaphase cells. d, Three-color images of microtubules and nuclei in telophase cells. Representative images were from three independent samples. Scale bars, 20 µm.

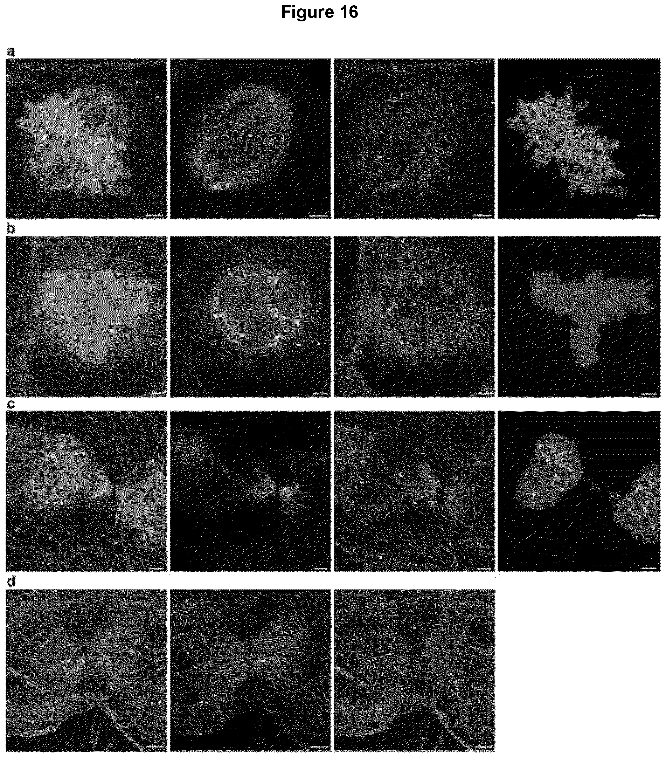

Figure 16: Comparison of TREx-expanded microtubules in mitotic COS-7 cells using compound 1b (inventive example 1) and antibodies to α tubulin (comparative example 2b). Microtubules stained with compound 1b (CF568, red) or with anti-α tubulin antibodies (AF 488, green) and nuclei stained with DAPI (cyan) are shown. a,b Three-color images of microtubules and nuclei in metaphase cells. c,d Three-color images of microtubules and nuclei in telophase cells. Representative images were from four independent samples. Scale bars, 20 µm.

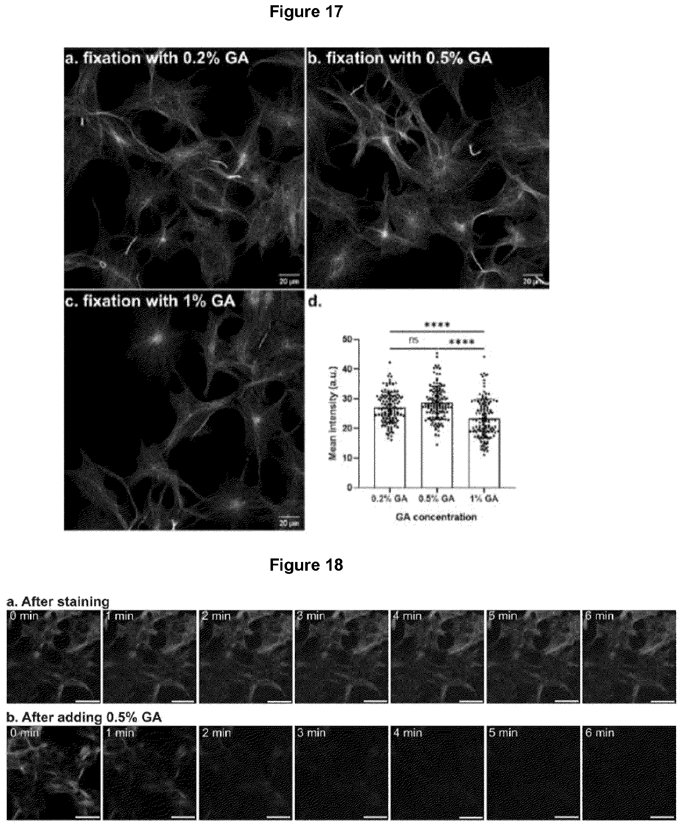

Figure 17: Comparison of average fluorescence intensity of microtubules obtained after fixation with different concentrations of GA. Specimens labeled with compound 1b (inventive example 1) was fixed, permeabilized and click labeled with DBCO-modified Alexa 594. a-c, Representative images of microtubules fixed with 0.2% (a), 0.5% (b) and 1% GA (c). d, Average fluorescence intensity of microtubules achieved after fixation. Bars represent the mean value and error bars represent the standard deviation. Statistical significance was assessed by one-way ANOVA test. **** p < 0.0001, ns (non-significant) = 0.0613. From left to right, mean values are 27.10 ± 5.11 (mean ± standard deviation, n =132 from three independent samples), 28.72 ± 5.65, 23.39 ± 6.55, respectively. Representative images from n=3 independent samples.
Figure 18: Evaluation of the fixability of SiR-tubulin (comparative example 1). a, Time-lapse recording of fluorescent signal of SiR-tubulin-labeled microtubules in living COS-7 cells. b, Time-lapse recording of fluorescent signal of microtubules stained with SiR-tubulin after fixing cells with 0.5% GA. Representative images were from two independent samples. Scale bars, 30 µm.

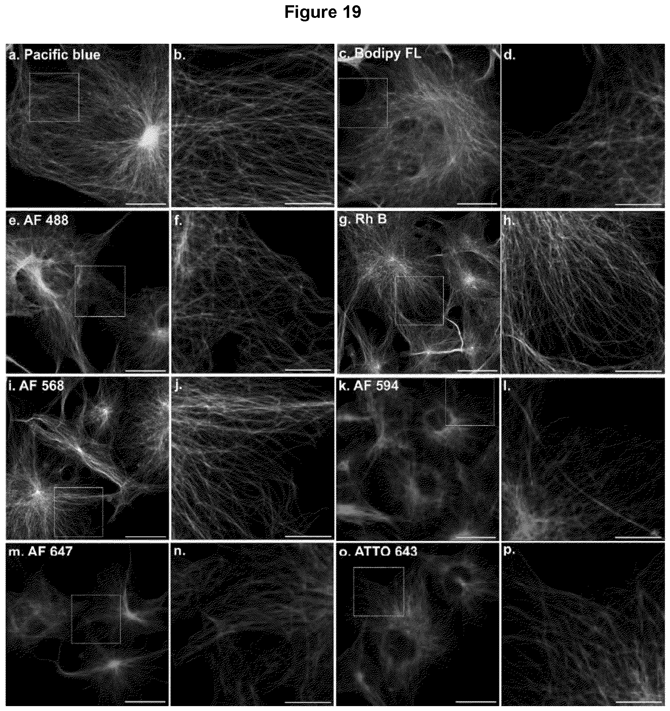

Figure 19: Airyscan microscopy images of microtubules in COS-7 cells with probe 1b (inventive example 1) and various organic dyes as labeling compound. a,b, Airyscan SR image of microtubules with Pacific blue. c,d, Airyscan SR image of microtubules with Bodipy FL. e,f, Airyscan SR image of microtubules with AF 488. g,h, Airyscan SR image of microtubules with Rhodamine B. i,j, Airyscan SR image of microtubules with AF 568. k,l, Airyscan SR image of microtubules with AF 594. m,n, Airyscan SR image of microtubules with AF 647. o,p, Airyscan SR image of microtubules with ATTO 643. Representative images were from three independent samples. Scale bars, 30 µm (a,c,e,g,i,k,m,o), and 10 µm (b,d,f,h,j,l,n,p).

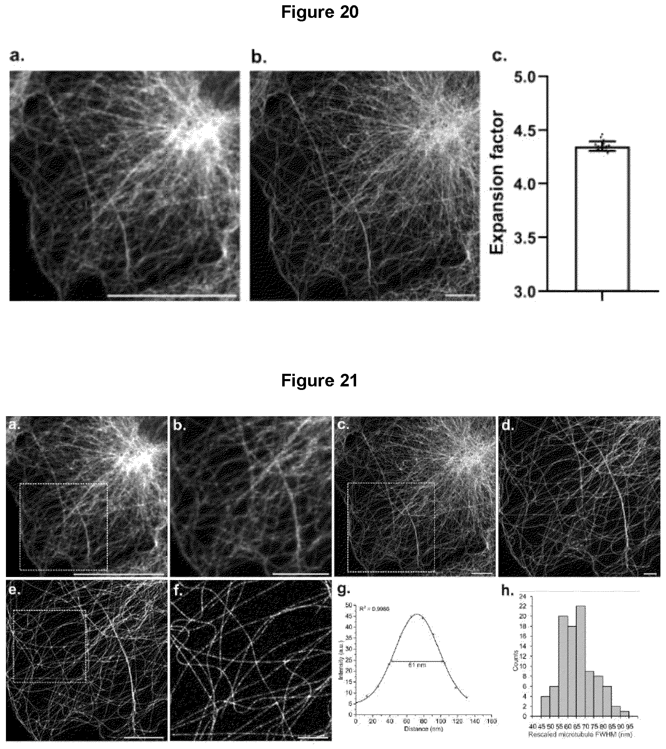

Figure 20: Expansion factor determination using the original ExM protocol (4xExM) on COS-7 cells labeled with compound 1b (inventive example 1), fixed and permeabilized, followed by click-reaction with DBCO-biotin as labeling compound and staining with CF568-modified streptavidin. a, Representative image of microtubules before expansion. b, Post-expansion image of the same microtubules. c, Analysis of expansion factor obtained after 4x ExM, yielding an expansion factor of 4.3 ± 0.046 (mean ± standard deviation, n= 18). Scale bars, 30 µm.
Figure 21: Microtubules of the same group of samples as shown in Figure 20 visualized by 4x-ExM-Airyscan SR. a-f, Comparison of pre- and post-expansion images of microtubules (CF 568) in cultured COS-7 cells in 4x ExM. a, Pre-expansion confocal fluorescence image of microtubules in 4x ExM. b, Magnified views of the boxed region in panel a. c, Post-expansion confocal fluorescence image of microtubules in the same cell. d, Magnified views of the boxed region in panel c. e, Airyscan SR image of the same area in panel d. f, Zoom-in views of the highlighted region in panel e. g, A representative cross-sectional intensity profile of microtubules (red dots) with Gaussian fitting (solid line). h, Distribution of Gaussian-fitted full width at half-maximum (FWHM) of 96 microtubule intensity profiles, yielding a resolution of 65.68 ± 9.54 nm (mean ± s.d.). Representative images were from two independent samples. Scale bars: 30 µm (a,c,e), and 10 µm (b,d,f).

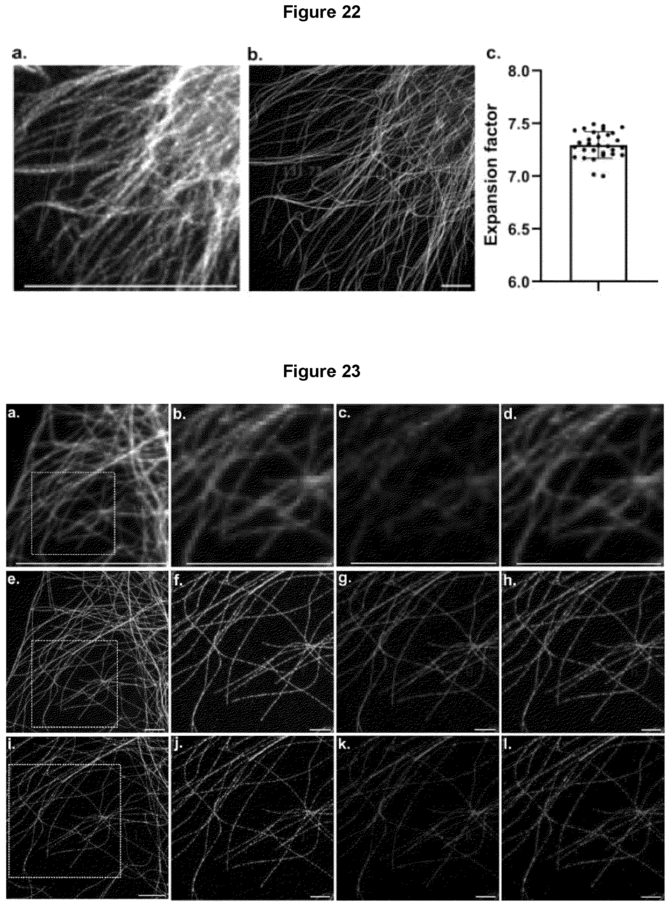

Figure 22: Expansion factor calculation for microtubules labeled as the samples shown in Figure 20 expanded using the TREx protocol. a, Representative image of microtubules before expansion. b, Post-expansion image of the same microtubules. c, Analysis of expansion factor obtained after TREx, yielding an expansion factor of 7.3 ± 0.13 (mean ± standard deviation, n= 30). Scale bar, 30 µm.
Figure 23: Multi-color images of microtubules stained with compound 1b (inventive example 1) and anti-β-tubulin antibodies (comparative example 2a) in TREx. a, Pre-expansion confocal fluorescence image of microtubules visualized with compound 1b (CF 568, red) or immunostained β-tubulin (AF 488, green). b, Magnified views of the boxed region in panel a. c,d, Red and green channel in panel b. e, Post-expansion confocal fluorescence images of microtubules obtained after TREx in the same cell. f, Magnified views of the boxed region in panel e. g,h, Red and green channel in panel f. i, Airyscan SR image of the same cell after TREx. j, Magnified views of the boxed region in panel i. k,l, Red and green channel in panel j. Representative images were from three independent samples. Scale bars, 20 µm (a,e,i) and 10 µm (b-d,f-h,j-l).

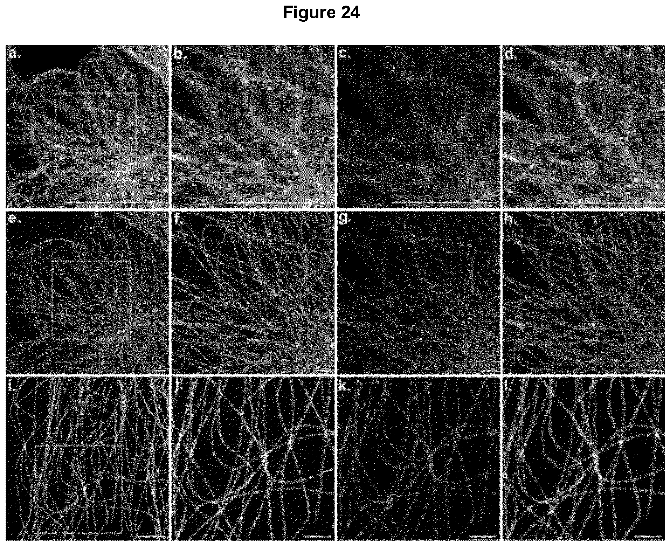

Figure 24: Multi-color images of microtubules stained with compound 1b (inventive example 1) and anti-α-tubulin antibodies (comparative example 2b) in TREx. a, Pre-expansion confocal fluorescence image of microtubules visualized with compound 1b (CF 568, red) or immunostained α-tubulin (AF 488, green). b, Magnified views of the boxed region in panel a. c,d Red and green channel in panel b. e, Post-expansion confocal fluorescence image of microtubules obtained after TREx in the same cell. f, Magnified views of the boxed region in panel e. g,h Red and green channel in panel f. i, Airyscan SR image of microtubules in different cells after TREx. j Magnified views of the boxed region in panel i. k,l, Red and green channel in panel j. Representative images were from three independent samples. Scale bars: 20 µm (a,e,i), and 10 µm (b-d,f-h,j-l).

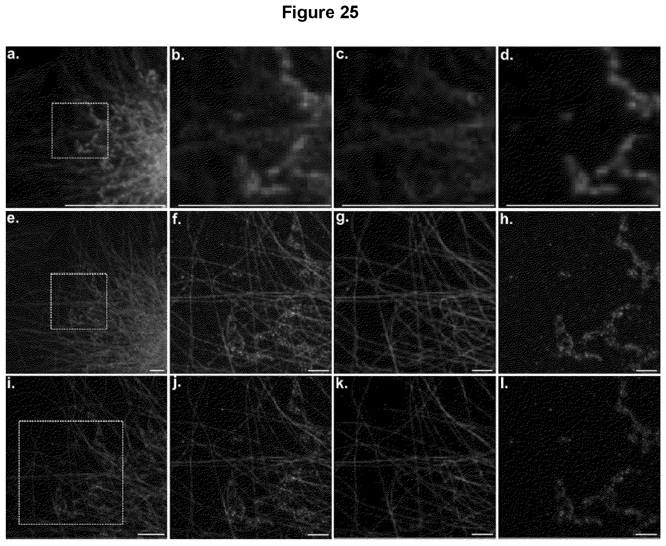

Figure 25: Multi-color images of compound 1b (inventive example 1)-labeled microtubules and immunostained mitochondria in TREx. a, Pre-expansion confocal fluorescence image of microtubules visualized with compound 1b (CF 568, red) or immunostained mitochondria (AF 488, TOMM20). b, Magnified views of the boxed region in panel a. c,d, Red and green channel in panel b. e, Post-expansion confocal fluorescence image of microtubules obtained after TREx in the same cell. f, Magnified views of the boxed region in panel e. g,h Red and green channel in panel f. i, Airyscan SR image of the same cell after TREx. j, Magnified views of the boxed region in panel i. k,l, Red and green channel in panel j. Representative images were from three independent samples Scale bars, 20 µm (a,e,i), and 10 µm (b-d,f-h,j-l).

### EXAMPLE SECTION

### 1) Methods

### a) Synthesis and characterization of compounds

Chemicals were purchased from Sigma-Aldrich, TCI, ACROS or Jena Bioscience. Dry solvents were used without further purification. Reactions were monitored with thin layer chromatography (TLC) with silica gel plates (Kieselgel 60 F254 plates, Merck) under UV light. 70-230 mesh silica 60 (E. M. Merck) was used to purify compounds on column chromatography. Mass spectra was obtained on a Shimadzu LC-MS 2020 Liquid Chromatograph Mass Spectrometer (ShimPack Gist C18 2 µm, 2.1x100 mm). ¹H NMR and ¹³C NMR spectra were acquired on a Bruker Avance 400 MHz or a Bruker Avance II + 600 MHz spectrometer using MeOD-d₄ or CDCl₃ as a solvent.

### b) Cell culture

COS-7 cells were cultured in DMEM/HAM's F12 medium supplemented with 10% fetal bovine serum (FBS) and 100 U/mL penicillin and 0.1 mg/mL streptomycin at 37 °C in a humidified 5% CO₂ incubator. Cells were seeded in 4-well Cellvis (Cellvis, # C 4-1.5H-N) at a concentration of 3×10⁴ cells/well or on a 12 mm coverslip (high precision, No. 1.5H) in 4-well plates at a concentration of 7×10⁴ cells/well for expansion experiments. Cells were then incubated for 24 h before using.

### c) Microtubule staining with SiR-tubulin in living cells

The staining is performed based on instructions in the manufacturer's manual. COS-7 cells were incubated with 1 µM SiR-tubulin (comparative example 1) in DMEM medium containing 10 µM verapamil and 1 µg/mL Hoechst 33342 for 1 h at 37 °C. Cells were then washed once with DMEM medium containing 10 µM verapamil prior to imaging.

### d) Microtubule staining with fixable docetaxel probes

COS-7 cells were fed with 3 µM of the docetaxel probe 1b (inventive example 1) in DMEM medium for 1 h at 37 °C. Cells were then fixed with 0.5% GA in PEM solution (80 mM PIPES pH 6.8, 5 mM EGTA,1 mM MgCl2) containing 0.3% Triton X-100 for 10 min. After rinsing with PBS, cells were quenched with freshly prepared 0.15% sodium borohydride in PBS at room temperature for 7 min. Cells were washed three times with PBS and treated with 20 mM N-ethylmaleimide for 30 min to to block free thiols. Samples were further washed three times with PBS. Afterwards, stained samples were incubated with 10 µM DBCO-modified dyes in PBS containing 1% BSA, followed by washing three times with PBS containing 0.1% Tween-20. For samples used in ExM, after blocking free thiols with N-ethylmaleimide, samples were with 10 µM Biotin-PEG4-DBCO in PBS containing 1% BSA, followed by washing three times with PBS containing 0.1% Tween-20. Samples were then stained with 15 µg/mL CF568-modified streptavidin in PBS containing 1% BSA for 1 h and washed three times with PBS. Finally, cells were then stained with 1 µg/mL DAPI for 1 min and washed three times with PBS prior to imaging.

### e) Optimization of microtubule staining for dSTORM

After incubating cells with 3 µM of the docetaxel probe (inventive example 1) in DMEM medium for 1 h at 37 °C, cells were fixed as mentioned above. Samples were treated with 20 mM N-ethylmaleimide and washed three times with PBS. Samples were then stained with 2 µM DBCO-AF 647 in PBS at room temperature for 50 min, followed by washing three times with PBS containing 0.1 % Tween-20. Next, samples were incubated with 5% BSA for 20 min and immediately post-fixed with 4% PFA for 10 min. Samples were then rinsed three times with PBS before image acquisition.

### f) Multi-color staining

For co-staining with antibodies to α/β tubulin (comparative examples 2b and 2a), after docetaxel-microtubule staining, cells were blocked twice with blocking buffer (PBS, 1% BSA) for 5 min. Cells were incubated with 5 µg/mL primary antibody (rabbit anti α-tubulin, Abcam, ab18251 or mouse anti-β tubulin, Merck, T8328) in staining buffer (PBS, 3 % BSA) for 1 h at room temperature and washed three times with PBS. Cells were then incubated with AF 488-labeled secondary antibodies (Goat Anti-Rabbit F(ab)2 IgG, ThermoFisher, A-11070 or Goat Anti-Mouse IgG, Abcam, ab150113) in staining buffer for 1 h with a dilution of 100x. After rinsing twice with PBS, samples were washed twice with blocking buffer. Samples were then stained with 1 µg/mL DAPI for 1 min and washed three times again with PBS prior to imaging. For co-staining with antibodies to mitochondria, after incubating cells with the docetaxel probe, cells were fixed with 0.2% GA in PEM solution containing 0.1% Triton X-100 for 10 min. Cells were then rinsed once with PBS and quenched with 0.15% sodium borohydride in PBS for 7 min. After washing three times with PBS, cells were further permeabilized with 0.2% Triton X-100 for 10 min and washed three times with PBS. Cells were stained with CF568-modified streptavidin as abovementioned. Next, samples were then blocked with permeabilization/blocking buffer (PBS, 3% BSA and 0.25% Triton X-100) for 20 min, followed by staining with primary antibodies (rabbit anti-TOMM20, Abcam, ab186734) in staining buffer at a concentration of 4 µg/mL for 1 h at room temperature. After washing three times with PBS, samples were then incubated with AF 488-labeled secondary antibodies (Goat Anti-Rabbit F(ab)2 IgG, ThermoFisher, A-11070) in staining buffer for 1 h with a dilution of 100x. Samples were then rinsed twice with PBS and washed twice with blocking buffer. Finally, samples were stained with DAPI as mentioned above. For co-staining with actin filaments, the docetaxel-microtubule staining was performed as mentioned above, except that microtubules were labeled with 10 µg/mLATTO 643-streptavidin. Next, actin filaments were stained with the trifunctional phalloidin linker (the 'Actin ExM' kit, fluorophore 561) in PBS at a concentration of 0.5 µM at room temperature for 1 h, followed by washing three times with PBS. Samples were then stained with DAPI prior to imaging.

### g) Gelation, digestion and expansion

The procedure of ExM was performed as previously described with some modifications (Chen, F., Tillberg, P.W & Boyden, E.S. Expansion microscopy. Science 347, 543-548 (2015); Damstra, H.G. et al. Visualizing cellular and tissue ultrastructure using Ten-fold Robust Expansion Microscopy (TREx). Elife 11, e73775 (2022)). For 4x ExM, samples were first incubated with 0.1 mg/mL AcX in PBS for 90 min at room temperature and washed three times with PBS for 5 min. Afterwards, samples were quickly rinsed with 50 µL monomer solution (2 M NaCl, 8.625% (w/w) sodium acrylate, 2.5% (w/w) acrylamide, 0.15% (w/w) N,N'-methylenebisacrylamide and 0.01% 4-hydroxy-TEMPO in PBS) containing tetramethylenediamine (TEMED, 0.15% w/w) and ammonium persulfate (APS, 0.15% w/w), followed by polymerization on the gelation chamber with another 50 µL gelation solution in a humidified incubator at 37 °C for 1.5 h. Samples were then digested with proteinase K (New England Biolabs) at a concentration of 8 units/mL in digestion buffer (50 mM Tris (pH 8.0), 1 mM EDTA, 0.5% TritonX-100, 0.8 M guanidine HCl) at 37 °C for 3 h.

For TREx, samples were incubated with 0.1 mg/mL AcX in PBS containing 200 mM NaHCO3 for 90 min at room temperature and washed three times with PBS for 5 min prior to the gelation. The stained samples were quickly rinsed with 50 µL monomer solution (1.1 M sodium acrylate, 2.0 M acrylamide, 90 µg/mL N,N'-methylenebisacrylamide in PBS) supplemented with 1.5 mg/mLAPS, 1.5 mg/mL TEMED, and 15 µg/mL 4-hydroxy TEMPO, followed by polymerization on the gelation chamber with another 50 µL gelation solution in a humidified incubator at 37 °C for 1 h. Samples were then digested with proteinase K (New England Biolabs) at a concentration of 8 units/mL in digestion buffer (50 mM Tris (pH 8.0), 1 mM EDTA, 0.5% TritonX-100, 0.8 M guanidine HCl) at room temperature for 10 h. Samples were digested with proteinase K (New England Biolabs) at a concentration of 8 units/mL in digestion buffer (50 mM Tris (pH 8.0), 2 mM CaCl2,0.5% TritonX-100, and 0.8 M guanidine HCl) at 37 °C for 10 h when the co-staining with antibodies to anti-α/β tubulin was performed. The digested samples were re-stained with 1 µg/mL DAPI in PBS for 1 min and washed three times with 1x PBS. Finally, the digested gels were expanded with double-deionized H2O for 10 minutes. This expansion step was repeated four times with H2O until gels were fully expanded.

### h) Microscopy and image analysis

Airyscan confocal or SR images were acquired on a LSM 900 with Airyscan 2 (Zeiss) system using a C-Apochromat 40x/1.2 numerical aperture (NA) water-immersion objective. For various organic dyes applied in the paper, excitation wavelengths and filter settings for the respective dyes were chose with the integrated dye presets in the ZEN 2 blue software (version 3.5) from Zeiss. The standard strength mode of 3D Airyscan processing was used to process the Airyscan SR data. SIM images were acquired on an Elyra 7 (Zeiss) equipped with four excitation lasers, a 405 nm diode (50 mW), a 488 nm OPSL (500 mW), a 561 nm OPSL (500 mW) and a 642 nm diode laser (500 mW) using a Plan-Apochromat 63x/1.4 NA oil DIC M27 objective. Laser emissions were filtered by band pass (BP) 570-620, long pass (LP) 655, BP 420-480, or BP 495-550. SIM processing was performed with the Zeiss ZEN 3.0 SR FP2 (black) software. dSTORM images were recorded on a homebuilt widefield setup with an inverted microscope (Olympus IX-71) using an oil immersion objective (Olympus APON 60xO TIRF, NA 1.49). The system is equipped with excitation lasers of the wavelengths 639 nm (Genesis MX639-1000, Coherent), 558 (Genesis MX561-500, Coherent), 514 nm (Genesis MX514-500, Coherent) and 405 nm (iBeam-smart-S 405-S, TOPTICA). To separate the excitation beam from the fluorescence, a dichroic beam splitter was applied (ZT405/514/635rpc) and the emission was additionally filtered by an emission filter (Brightline HC 679/41 (Semrock) in front of the EMCCD-cameras (iXon Ultra 897, Andor). dSTORM imaging was acquired in a 100 mM MEA buffer (pH 7.4) with integration times of 20 ms for 30,000 frames at laser intensities of ~3 kW/cm² and additional pulsing with UV. Acquired image stacks were reconstructed with rapidSTORM 3.3 (Wolter, S. et al. rapidSTORM: accurate, fast open-source software for localization microscopy. Nat. Methods 9, 1040-1041 (2012)). After image acquisition, all Airyscan confocal and SR data were analysed with ImageJ (Schindelin, J. et al. Fiji: an open-source platform for biological-image analysis. Nat. Methods 9, 676-682 (2012)). Expansion factors were determined by comparing the same features of COS-7 cells before and after expansion. The image resolution was measured with ImageJ 'FWHM_line' plugin with the Gaussian fitting and recalculated by expansion factor.

### 2) Examples

### a) Comparative example 1 (CE1):

Microtubule staining was performed with commercially available SiR-tubulin consisting of a docetaxel moiety directly conjugated with a fluorogenic dye.

### b) Comparative example 2 (CE2):

Microtubule staining was performed with anti-β-tubulin (comparative example 2a) or anti-α-tubulin antibodies (comparative example 2b).

### c) Comparative example 3 (CE3):

The probe synthesized and used for staining as comparative example 3 is a docetaxel derivative according to compound 1a. The docetaxel derivative of compound 1a as shown below is modified at the nitrogen atom to comprise a derivatization comprising an alkynyl but no primary amino-group.

### Synthesis of compound 1a

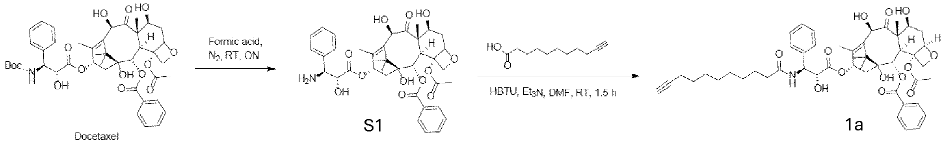

Docetaxel (100 mg) was dissolved in 0.5 mL formic acid and the reaction mixture was stirred for 3 h at room temperature. After complete reaction, all solvents were evaporated to yield the intermediate (compound S1) as a white solid, which was used without further purification.

Characterization of compound S1: **LC-MS** (ESI⁺): calculated for C₃₈H₄₆NO₁₂ [M+H]⁺ m/z: 708.30; found: 708.10.

To a solution of 10-undecynoic acid (11.5 mg, 63.4 µmol) in DMF (0.7 mL), triethylamine (26.4 µL, 190 µmol), HBTU (26.4 mg, 69.7 µmol) and the modified docetaxel intermediate (52.5 mg, 69.7 µmol) were added and the reaction mixture was stirred at room temperature for 1.5 h. After complete reaction, 30 mL ethyl acetate was added into the reaction flask, followed by washing with water (30 mL, 2 x) and brine (30 mL). The organic layer was dried over MgSO4 and then evaporated under reduced pressure. The residue was purified by column chromatography to yield the product as a white solid (56%).

Characterization of compound 1a: **LC-MS** (ESI⁺): calculated for C₄₉H₆₁NO₁₃Na [M+Na]⁺ m/z: 894.40; found: 894.15; **¹H NMR** (400 MHz, MeOD-d₄) δ 8.14 - 8.07 (m, 2H), 7.66 (t, J = 7.4 Hz, 1H), 7.56 (t, J = 7.6 Hz, 2H), 7.47 - 7.36 (m, 4H), 7.33 - 7.24 (m, 1H), 6.15 (t, J = 8.6 Hz, 1H), 5.65 (d, J = 7.2 Hz, 1H), 5.46 (d, J = 4.5 Hz, 1H), 5.27 (s, 1H), 5.03 - 4.96 (m, 1H), 4.58 (d, J = 4.6 Hz, 1H), 4.31 - 4.13 (m, 3H), 3.87 (d, J = 7.2 Hz, 1H), 2.51 - 2.40 (m, 1H), 2.34 (s, 3H), 2.31 - 2.19 (m, 3H), 2.19 - 2.11 (m, 3H), 2.02 (dd, J = 15.4, 8.8 Hz, 1H), 1.90 (d, J = 0.9 Hz, 3H), 1.88 - 1.79 (m, 1H), 1.70 (s, 3H), 1.65 - 1.55 (m, 2H), 1.52 - 1.43 (m, 2H), 1.43 - 1.34 (m, 2H), 1.34 - 1.25 (m, 6H), 1.19 (s, 3H), 1.13 (s, 3H); **¹³C NMR** (101 MHz, MeOD-d₄) δ 209.18, 174.17, 172.51, 169.89, 165.79, 138.26, 137.33, 136.09, 132.63, 129.53, 129.29, 127.78, 127.74, 126.96, 126.52, 84.02, 83.20, 80.46, 77.16, 75.71, 74.55, 73.76, 72.93, 70.77, 70.69, 67.49, 56.98, 54.92, 45.99, 42.64, 35.61, 35.14, 34.92, 28.43, 28.32, 28.15, 27.82, 27.78, 25.21, 25.19, 21.32, 19.77, 17.10, 12.51, 8.56. NMR spectra shown in Figure 1.

### d) Inventive example 1 (IE1):

A docetaxel derivative according to compound 1b, the derivatization introducing a primary amino- and an azido-group, was synthesized and used for microtubule staining. Compound 1b is herein further referred to as inventive example 1.

### Synthesis of compound 1b

First, compound S2 was synthesized. To an ice-cold solution of 6-aminohexanoic (7.87 g, 60 mmol) in 2N NaOH (75 mL, 150 mmol) was added a solution of tert-butyl dicarbonate (14.4 g, 66 mmol) in dioxane (75 mL) in portion. Then the reaction mixture was stirred at 0 °C for 1 h. After completion of the reaction, the dioxane was removed by evaporation. The aqueous layer was washed with diethyl ether and acidified by addition of saturated KHSO₄ (aq) to pH 2-3, followed by extraction of EtOAc (80 mL, 3 x). The combined organic layer was washed with brine (80 mL), dried over anhydrous Na₂SO₄, filtered and concentrated to yield the product S2 as a white foam (13.2 g, 95% yield).

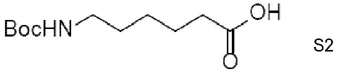

Characterization of compound S2: **LC-MS** (ESI⁻): calculated for C₁₁H₂₀NO₄⁻ [M-H]⁻ m/z: 230.14; found: 229.90; ¹H **NMR** (600 MHz, CDCl₃) δ 4.60 (s, 1H), 3.13 (d, J = 6.0 Hz, 2H), 2.36 (t, J = 7.4 Hz, 2H), 1.70 - 1.63 (m, 2H), 1.52 (dd, J = 14.8, 7.4 Hz, 2H), 1.45 (s, 9H), 1.41 - 1.35 (m, 2H). NMR spectrum is shown in Figure 2.

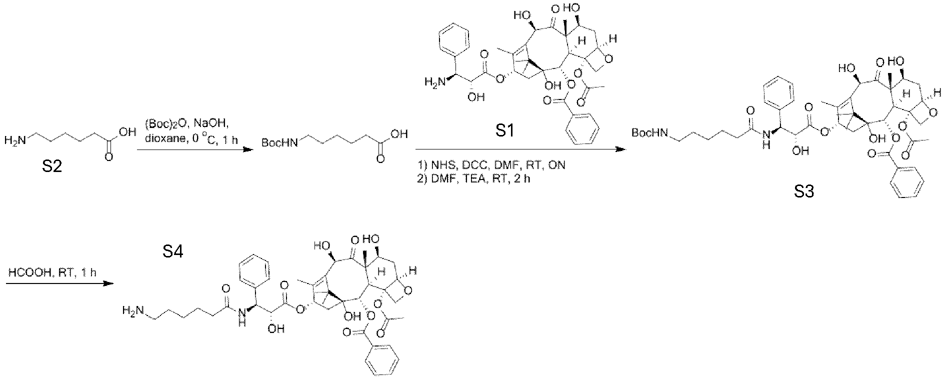

To a solution of S2 (1.85 g, 8 mmol) in DMF (10 mL) were added DCC (4.95 g, 24 mmol) and N-Hydroxysuccinimide (2.76 g, 24 mmol). Then the reaction mixture was stirred at room temperature overnight. After completion of the reaction, the reaction mixture was filtered and the filtrate was evaporated. The crude was resuspended in EtOAc and stored in the freezer for 1 h, followed by filtration, evaporation and drying by high vacuum. The NHS ester product was obtained as a white solid and used for next step directly without further purification. Subsequently, to a solution of S1 (175 mg, 0.247 mmol) in DMF (4 mL) were added the NHS ester (162 mg, 0.494 mmol) obtained above and triethylamine (103 µL, 0.741 mmol). The reaction mixture was stirred at room temperature for 2 h. After completion of the reaction, the reaction mixture was filtered and water (40 mL) was added into the filtrate, followed by extraction of EtOAc (20 mL, 3 x). The combined organic layer was washed with brine (40 mL), dried over anhydrous Na₂SO₄, filtered and concentrated under reduced pressure. The residue was purified by column chromatography to yield the product S3 as a white solid (182 mg, 80% yield).

Characterization of compound S3: **LC-MS** (ESI⁺): calculated for C₄₉H₆₄N₂O₁₅Na⁺ [M+Na]⁺ m/z: 943.42; found: 943.25; **¹H NMR** (600 MHz, MeOD-d₄) δ 8.14 - 8.11 (m, 2H), 7.70 - 7.66 (m, 1H), 7.58 (t, J = 7.8 Hz, 2H), 7.45 - 7.40 (m, 4H), 7.31 - 7.28 (m, 1H), 6.18 (t, J = 8.6 Hz, 1H), 5.67 (d, J = 7.2 Hz, 1H), 5.47 (d, J = 4.7

Hz, 1H), 5.29 (s, 1H), 5.01 (dd, J = 9.6, 1.8 Hz, 1H), 4.60 (s, 1H), 4.24 (dd, J = 11.3, 6.6 Hz, 1H), 4.21 (t, J = 5.8 Hz, 2H), 3.89 (d, J = 7.2 Hz, 1H), 3.00 (t, J = 7.1 Hz, 2H), 2.46 (ddd, J = 14.5, 9.8, 6.7 Hz, 1H), 2.36 (s, 3H), 2.31 (td, J = 7.3, 4.6 Hz, 2H), 2.26 (dd, J = 15.3, 9.4 Hz, 1H), 2.06 - 1.99 (m, 1H), 1.92 (s, 3H), 1.85 (ddd, J = 13.9, 11.3, 2.2 Hz, 1H), 1.72 (s, 3H), 1.66 - 1.59 (m, 2H), 1.44 (s, 9H), 1.37 - 1.29 (m, 4H), 1.21 (s, 3H), 1.15 (s, 3H). ¹³C **NMR** (151 MHz, MeOD-d₄) δ 4209.72, 174.51, 173.04, 170.42, 166.30, 157.13, 138.73, 137.83, 136.58, 133.16, 130.03, 129.79, 128.30, 128.26, 127.49, 127.02, 84.53, 80.95, 78.43, 77.70, 76.22, 75.04, 74.26, 73.47, 71.27, 71.15, 57.49, 55.49, 46.48, 43.15, 39.79, 36.11, 35.52, 35.43, 29.23, 27.41, 26.03, 25.70, 25.35, 21.83, 20.30, 13.03, 9.08. NMR spectra are shown in Figure 3.

S3 (92 mg, 0.1 mmol) was dissolved in 0.5 mL formic acid and the reaction mixture was stirred for 1 h at room temperature. After completion of the reaction, all solvents were evaporated to yield the intermediate S4 as a white solid (100% yield), which was used without further purification.

Characterization of compound S4: **LC-MS** (ESI⁺): calculated for C₄₄H₅₇N₂O₁₃⁺ [M+H]⁺ m/z: 821.39; found: 821.20.

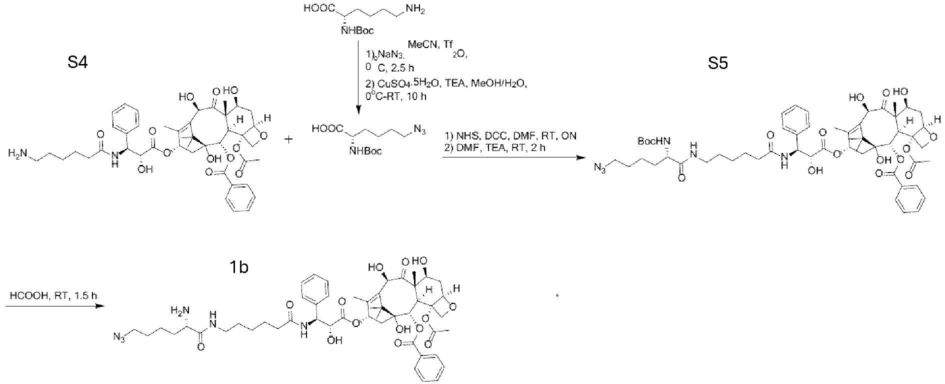

As described in the literature (Jain, D. R.; Ganesh, K. N. Clickable Cγ-Azido(Methylene/Butylene) Peptide Nucleic Acids and Their Clicked Fluorescent Derivatives: Synthesis, DNA Hybridization Properties, and Cell Penetration Studies. J. Org. Chem. 2014, 79 (14), 6708-6714.), to a suspension of NaN₃ (380 mg, 5.85 mmol) in MeCN (5 mL) was added triflic anhydride (820 µL, 4.87 mmol) in a dropwise manner in an ice bath. Then the reaction mixture was stirred at 0 °C for 2.5 h to form triflyl azide. Subsequently, the obtained triflyl azide was diluted with CH₂Cl₂ (35 mL). To a stirred solution of Boc-Lys-OH (1.0 g, 4.06 mmol) in MeOH/water mixture (15 mL/15 mL) were added triethylamine (1.69 mL, 12.18 mmol) and CuSO₄·5H₂O (50.7 mg, 0.2 mmol, in 0.5 mL H₂O), followed by addition of the diluted triflyl azide in CH₂Cl₂ through a dropping funnel. Then the reaction mixture was stirred at room temperature for 10 h. After completion of the reaction, the solvent was evaporated and saturated NaHCO3 (40 mL) was added to the residue, followed by extraction of EtOAc (30 mL, 3 x). The aqueous layer was acidified by slow addition of saturated KHSO4(aq) to pH 2-3 and extracted with EtOAc (40 mL, 3 x). The combined organic layer was washed with brine (80 mL), dried over anhydrous Na2SO4, filtered and concentrated under reduced pressure to give N²-(tert-butoxycarbonyl)-N⁶-diazo-L-lysine as a yellow oil, which was used to make NHS ester without further purification.

To a solution of N²-(tert-butoxycarbonyl)-N⁶-diazo-L-lysine obtained above in DMF (8 mL) were added DCC (2.26 g, 11 mmol) and N-Hydroxysuccinimide (1.26 g, 11 mmol). Then the reaction mixture was stirred at room temperature overnight. After completion of the reaction, the reaction mixture was filtered and the filtrate was evaporated. The crude was resuspended in EtOAc and stored in the freezer for 1 h, followed by filtration, evaporation and drying by high vacuum. The NHS ester product was obtained as a white solid and used for next step directly without further purification. Subsequently, to a solution of S4 (82 mg, 0.1 mmol) in DMF (2 mL) were added the NHS ester (111 mg, 0.3 mmol) obtained above and triethylamine (41.7 µL, 0.3 mmol). The reaction mixture was stirred at room temperature for 2 h. After completion of the reaction, the reaction mixture was filtered and the filtrate was concentrated under reduced pressure. The residue was purified by column chromatography to yield the product S5 as a white solid (67 mg, 62% yield).

Characterization of compound S5: **LC-MS** (ESI⁺): calculated for C₅₅H₇₅N₆O₁₆⁺ [M+H]⁺ m/z: 1075.52; found: 1075.65; **¹H NMR** (400 MHz, MeOD-d₄) δ 8.17 - 8.09 (m, 2H), 7.70 - 7.65 (m, 1H), 7.58 (t, J = 7.6 Hz, 2H), 7.47 - 7.37 (m, 4H), 7.33 - 7.26 (m, 1H), 6.18 (t, J = 8.6 Hz, 1H), 5.67 (d, J = 7.2 Hz, 1H), 5.52 - 5.44 (m, 1H), 5.29 (s, 1H), 5.17 (s, 4H), 5.01 (dd, J = 9.5, 1.7 Hz, 1H), 4.61 (d, J = 4.6 Hz, 1H), 4.29 - 4.17 (m, 3H), 3.99 (dd, J = 8.1, 5.7 Hz, 1H), 3.89 (d, J = 7.1 Hz, 1H), 3.30 (t, J = 6.7 Hz, 2H), 3.23 - 3.07 (m, 2H), 2.46 (ddd, J = 14.4, 9.7, 6.5 Hz, 1H), 2.36 (s, 3H), 2.34 - 2.20 (m, 3H), 2.04 (dd, J = 15.5, 8.8 Hz, 1H), 1.92 (d, J = 0.8 Hz, 3H), 1.85 (ddd, J = 13.8, 11.4, 2.1 Hz, 1H), 1.72 (s, 3H), 1.69 - 1.56 (m, 5H), 1.55 - 1.48 (m, 2H), 1.45 (s, 9H), 1.44 - 1.27 (m, 5H), 1.21 (s, 3H), 1.15 (s, 3H). **¹³C NMR** (101 MHz, MeOD-d4) δ 209.72, 174.41, 173.55, 173.05, 170.40, 166.28, 156.39, 138.75, 137.82, 136.59, 133.15, 130.04, 129.81, 128.31, 128.27, 127.49, 127.02, 84.53, 80.95, 79.24, 77.70, 76.23, 75.05, 74.26, 73.47, 71.28, 71.18, 57.49, 55.45, 54.57, 50.88, 46.48, 43.17, 38.77, 36.13, 35.45, 31.67, 28.64, 28.13, 27.36, 26.01, 25.75, 25.24, 22.81, 21.86, 20.33, 18.93, 13.07, 9.11. NMR spectra are shown in Figure 4.

S5 (37 mg, 38 µmol) was dissolved in 0.4 mL formic acid and the reaction mixture was stirred for 1.5 h at room temperature. After completion of the reaction, all solvents were evaporated and the product was dried by high vacuum to yield compound 1b as a white solid (100% yield).

Characterization of compound 1b: **LC-MS** (ESI⁺): calculated for C₅₀H₆₇N₆O₁₄⁺ [M+H]⁺ m/z: 975.47; found: 975.60. **¹H NMR** (600 MHz, MeOD-d₄) δ 8.13 (d, J = 7.3 Hz, 2H), 7.68 (t, J = 7.4 Hz, 1H), 7.58 (t, J = 7.7 Hz, 2H), 7.45 - 7.40 (m, 4H), 7.30 (t, J = 7.0 Hz, 1H), 6.17 (t, J = 8.7 Hz, 1H), 5.67 (d, J = 7.2 Hz, 1H), 5.48 (d, J = 4.6 Hz, 1H), 5.29 (s, 1H), 5.17 (s, 9H), 5.01 (d, J = 8.1 Hz, 1H), 4.61 (d, J = 4.7 Hz, 1H), 4.27 - 4.18 (m, 3H), 3.89 (d, J = 7.1 Hz, 1H), 3.78 (s, 1H), 3.34 (t, J = 4.9 Hz, 2H), 3.23 (t, J = 6.7 Hz, 2H), 2.46 (ddd, J = 14.7, 9.7, 6.6 Hz, 1H), 2.35 (s, 3H), 2.28 (ddd, J = 24.8, 15.0, 8.4 Hz, 3H), 2.06 - 2.01 (m, 1H), 1.92 (s, 3H), 1.85 (d, J = 6.4 Hz, 1H), 1.72 (s, 3H), 1.68 - 1.59 (m, 5H), 1.57 - 1.51 (m, 2H), 1.46 (dd, J = 13.5, 8.8 Hz, 3H), 1.40 - 1.35 (m, 2H), 1.21 (s, 3H), 1.15 (s, 3H). **¹³C NMR** (151 MHz, MeOD-d₄) δ 209.73, 174.38, 173.10, 170.39, 168.71, 166.96, 166.27, 138.73, 137.84, 136.58, 133.17, 130.02, 129.80, 128.31, 128.29, 127.51, 127.01, 84.51, 80.95, 77.71, 76.21, 75.03, 74.27, 73.46, 71.30, 71.19, 57.49, 55.51, 53.05, 50.62, 46.49, 43.15, 39.02, 36.12, 35.45, 35.37, 30.93, 28.54, 28.07, 26.05, 25.74, 25.15, 21.82, 20.31, 18.91, 13.07, 9.09 (little HCOOH left). NMR spectra are shown in Figure 5.

As shown by tests of compound 1b ( inventive example 1) in different experimental settings for labeling microtubules for detection by various imaging techniques, the inventive compound allows improved staining of dense microtubule structures, such as microtubules of the mitotic spindles in mitotic cells, compared to standard compounds known in the prior art, such as anti-tubulin antibodies (CE2a and CE2b) and SiR-tubulin (CE1), or docetaxel derivatives not comprising a primary amino-group (CE3) (Figure 6). Compound 1b (inventive example 1) is fixable, which is an advantage over conventional taxane probes for labeling microtubules such as SiR-tubulin (CE1) (Figures 17 and 18). Furthermore, the compound of inventive example 1 and conjugate derived from the compound are compatible with various super-resolution imaging techniques. In particular, superior performance of the compound is shown in SIM and dSTORM imaging (Figures 9 and 10). Furthermore, in combination with biotin-click labeling (biotin as labeling compound) and fluorophore-functionalized streptavidin, the compound is suitable to label microtubules for Expansion microscopy and excellent expansion factors can be obtained (Figures 7, 8, 14 and 20-22). This approach is also compatible with co-staining of the sample (Figures 11 and 25). Further tests of compound 1b (inventive example 1) in comparison to immunolabeling of microtubules with anti-β-tubulin (CE2a) or anti-α-tubulin antibodies (CE2b) demonstrate the superior performance of the inventive compound over antibody staining in airyscan microscopy and expansion microscopy (Figures 12, 13, 15, 16, 23 and 24)). Figure 19 shows that the compound of inventive example 1 is compatible with a broad variety of organic dyes as labeling compound.

All tested experimental settings show that the compound of inventive example 1 enables improved staining of dense microtubule structures in mitotic cells compared to standard immunolabeling. In combination with biotin click-labeling and fluorescently labeled streptavidin, the inventive compound shows superior performance for imaging microtubules with unprecedented spatial resolution. In addition, the inventive compound is compatible with other super-resolution microscopy methods such as SIM and dSTORM.

## Claims

1. A compound for labeling microtubules according to formula (I) 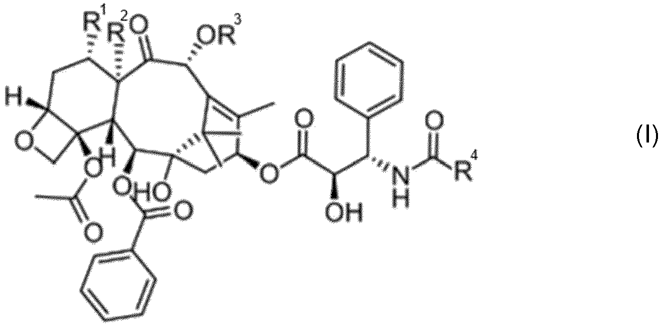 wherein R¹ represents a hydroxyl group, R² represents a methyl group and R³ represents a hydrogen atom or an acetyl group; or R¹ represents a methoxy group and R² and R³ represent a methyl group; or R¹ and R² are represented by formula (II) and R³ represents an acetyl group; and 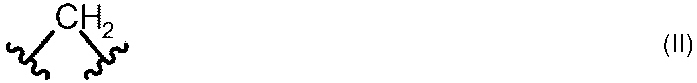 wherein R⁴ is represented by formula (III) 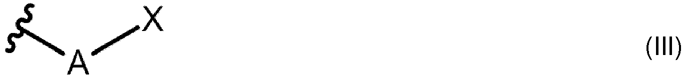
wherein A is a C₁ to C₂₅ alkyl substituted with at least one primary amino-group and one or more carbon atoms of the alkyl chain optionally being replaced by an amide group; and
wherein X is a group suitable for covalently linking the compound to form a conjugate with a labeling compound.

2. The compound according to claim 1, wherein R¹ represents a hydroxyl group, R² represents a methyl group and R³ represents a hydrogen atom; and/or A is represented by formula (IV) 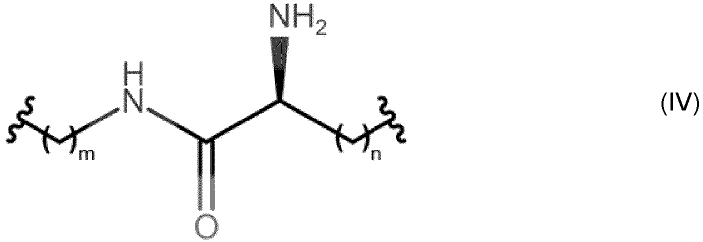 wherein m and n are numbers independently selected from the range of 1 to 10.

3. The compound according to claim 2, wherein m is a number from 3 to 7 and/or n is a number from 2 to 10.

4. The compound according to claim 3, wherein m is 5 and/or n is 4.

5. The compound according to any one of the preceding claims, wherein group X is an azido-group or an alkynyl-group, preferably an azido-group.

6. A conjugate formed by a reaction of compound (I) according to any one of claims 1 to 5 with a labeling compound comprising a group Y capable of reacting with group X of compound (I).

7. The conjugate according to claim 6, wherein group X is an azido-group and the group Y is an alkynyl-group.

8. The conjugate according to claims 6 or 7, wherein the labeling compound is a fluorophore functionalized with the group Y

9. The conjugate according to claims 6 or 7, wherein the labeling compound is an affinity-tag suitable to be further labeled by proteins or antibodies, preferably biotin, and functionalized with the group Y

10. A method for producing the compound according to any one of claims 1 to 5, wherein docetaxel, paclitaxel, cabazitaxel, or larotaxel is derivatized to the compound according to any one of claims 1 to 5.

11. Use of the compound (I) according to claims 1 to 5 and/or the conjugate according to any one of claims 6 to 9 in a method for labeling microtubules.

12. The use according to claim 11, wherein the conjugate is formed in situ.

13. The use according to claims 11 or 12, wherein the method for labeling microtubules comprises
I) contacting a sample with compound (I) to allow binding of said compound to the microtubules; and
II) contacting the sample having said compound bound to the microtubules with the labeling compound, wherein the conjugate is formed by a reaction of group X with group Y of the labeling compound.

14. The use according to any one of claims 11 to 13, wherein a labeling compound according to claim 9 is applied and the method further comprises
III) contacting the sample with a fluorophore-conjugated protein targeted to the labeling compound; and
IV) optionally further processing the sample for detecting the conjugate by Expansion Microscopy,
wherein preferably the labeling compound is biotin functionalized with the group Y and the protein targeted to the labeling compound is fluorophore-conjugated streptavidin.

15. A kit of parts for labeling microtubules comprising the compound (I) according to claim 1 to 5 and a labeling compound, wherein the labeling compound comprises a group Y capable of reacting with group X of compound (I) to form a conjugate.
